# EUROPEAN PATENT SPECIFICATION

(11) **EP 2 663 659 B1**
(45) Date of publication and mention of the grant of the patent: **19.10.2016**
(21) Application number: 12700271.5
(22) Date of filing: 10.01.2012
(51) Int. Cl.: C12R 1/425, A01H 3/00, A01H 17/00

(54) **SERRATIA PLYMUTHICA FOR BIOLOGICAL CONTROL OF BACTERIAL PLANT PATHOGENS**
SERRATIA PLYMUTHICA ZUR BIOLOGISCHEN BEKÄMPFUNG BAKTERIELLER PFLANZENPATHOGENE
SERRATIA PLYMUTHICA DANS LA RÉGULATION BIOLOGIQUE DE PATHOGÈNES DE VÉGÉTAUX BACTÉRIENS

(30) Priority: 11.01.2011 GB 201100427
(43) Date of publication of application: 20.11.2013
(73) Proprietor: Stichting Dienst Landbouwkundig Onderzoek, 6708 PB Wageningen (NL); Stichting voor de Technische Wetenschappen, 3527 JP Utrecht (NL)
(72) Inventor: VAN DER WOLF, Jean, Martin, 6708 LJ Wageningen (NL); CZAJKOWSKI, Robert, Lukasz, 81466 Gdynia (PL); VAN VEEN, Johannes, Antonie, 3911 AA Rhenen (NL)
(74) Representative: Williams, Richard Andrew Norman
(86) International application number: PCT/EP2012/050320
(87) International publication number: WO 2012/095431

(56) References cited:
- EP-A1- 1 241 247
- DATABASE WPI Week 200825 Thomson Scientific, London, GB; AN 2008-D47958 XP002671553, & KR 2007 0080775 A (IND ACADEMIC COOP FOUND GYEONGSANG NAT) 13 August 2007 (2007-08-13)
- R. CZAJKOWSKI ET AL: "Studies on the interaction between the biocontrol agent, Serratia plymuthica A30, and blackleg-causing Dickeya sp. (biovar 3) in potato (Solanum tuberosum)", PLANT PATHOLOGY, 1 November 2011 (2011-11-01), pages NO-NO, XP55021833, ISSN: 0032-0862, DOI: 10.1111/j.1365-3059.2011.02565.x
- JAFRA S ET AL: "Potential of bulb-associated bacteria for biocontrol of hyacinth soft rot caused by Dickeya zeae.", JOURNAL OF APPLIED MICROBIOLOGY JAN 2009 LNKD- PUBMED:19054227, vol. 106, no. 1, January 2009 (2009-01), pages 268-277, XP002671554, ISSN: 1365-2672
- DE VLEESSCHAUWER AND HÖFTE: "Using Serratia plymuthica to control fungal pathogens of plants", CAB REVIEWS, vol. 2, no. 46, 1 September 2007 (2007-09-01), pages 1-12, XP9157457,
- MONIKA SAA AWIAK ET AL: "Biochemical and genetical analysis reveal a new clade of biovar 3 Dickeya spp. strains isolated from potato in Europe", EUROPEAN JOURNAL OF PLANT PATHOLOGY, KLUWER ACADEMIC PUBLISHERS, DO, vol. 125, no. 2, 7 May 2009 (2009-05-07), pages 245-261, XP019728995, ISSN: 1573-8469, DOI: 10.1007/S10658-009-9479-2
- CZAJKOWSKI R ET AL: "Characterization of bacterial isolates from rotting potato tuber tissue showing antagonism to Dickeya sp. biovar 3 in vitro and in planta", PLANT PATHOLOGY (OXFORD), vol. 61, no. 1, February 2012 (2012-02), pages 169-182 URL, XP002671555,

## Description

The present invention concerns the field of biological control in agriculture and horticulture. In particular, the invention concerns biological control of bacterial plant pathogens, more particularly, the pathogens which are species of *Pectobacterium, Ralstonia* and *Dickeya* which cause soft rot, bacterial wilt or blackleg disease in potato.

Agricultural crops are susceptible to a large variety of microbial pathogens, which worldwide leads to enormous annual losses of produce and to concomitant economic damage. Methods developed to protect crops from plant diseases include plant breeding for resistance, cultural practices, application of chemical agents, and biological control.

Biological control of plant diseases generally is defined as suppression of pathogens by application of one or more organisms that exhibit antagonistic activity towards the pathogens. The organisms that act as antagonists usually are named biological control agents (BCAs). The mechanisms of the antagonistic effects are based on a variety of biological properties of BCAs. These comprise production of antibiotic compounds, expression of enzymes that catalyze the decomposition of cell components of pathogens, competition for space and nutrients, the ability to parasitize pathogens, and the induction of plant defense.

The application of chemical pesticides, which still prevails the management of plant diseases, generally exhibits negative side-effects on the environment and on human health. In addition, plant diseases relatively fast become resistant against chemical pesticides. This has increased the call for novel BCAs as safe and long-lasting alternative control agents.

Despite the well-documented efficacy of many different antagonists, only a limited number of strains are actually used in registered products directed to plant pathogenic microorganisms. Antagonists most commonly used in commercially available biocontrol products are the fungi *Trichoderma harzianum, Trichoderma polysporum* and *Gliacladium virens*, and the bacteria *Agrobacterium radiobacter, Pseudomonas fluorescens* and *Bacillus subtilis* (see Agrios G.N.,pages 322-328, in: Plant Pathology, 5th edition, 2004, Elsevier, Amsterdam). Examples of those products are F-Stop, BINAB^{®} T, Trichodex^{®}, GlioGard, Agrosin 84, Dagger G, and Kodiak.

The BCA products available are reported to control a broad spectrum of plant pathogenic microorganisms. Notably however, the pathogenic target organisms almost exclusively belong to the taxonomic kingdoms Protista and Fungi. Among these target organisms are various species of the genera *Pythium, Phytophthora, Botrytis, Fusarium, Rhizoctonia*, *Penicillium, Sclerotinia*, *Nectria* and powdery mildews. In contrast, only few bacterial pathogens are reported to be target for biocontrol products. Rare examples are *Agrobacterium tumefaciens*, responsible for crown gall disease (to be controlled by for example Agrosin 84), and *Erwinia amylovora,* responsible for fire blight in fruits (to be controlled by for example BlightBan^{®}).

The current commercial technology directed against bacterial plant pathogens is based mainly on the application of chemical pesticides (generally antibiotics).

The urgent need for microbial antagonists that effectively suppress bacterial plant pathogens is illustrated by the problematic management of potato soft rot and blackleg diseases, which are caused by species of *Pectobacterium* and *Dickeya.* These pectinolytic bacteria affect potato plants and tubers in virtually all phases of tuber production including storage, and form a continuous threat to (seed) potato production worldwide. Selection for blackleg and soft rot resistant potato cultivars has not resulted in seed lots free from *Pectobacterium* and *Dickeya* species (see Lapwood and Harris (1982), Potato Research 25, 41-50; Lapwood and Read (1984), Plant Pathology 33, 13-20). Physical (e. g. hot water treatment) and chemical procedures apparently disinfect only superficially and do not affect the high densities of pectinolytic *Pectobacterium* and *Dickeya* species that are located inside the vascular system at the stolon end of the tubers (see Czajkowski et al, (2009), European Journal of Plant Pathology 125, 263-275).

Blackleg and soft rot diseases caused by *Dickeya* and *Pectobacterium* species are causing major damage in seed potato production in Europe. The role of *Dickeya* spp. in the occurrence of potato blackleg seems to be increasing. This increase is associated with the detection of a new genetic clade of *Dickeya* spp., which could not be classified into one of the six species recently described by Samson et al. (2005, IJSEM 55: 1415-1427) (See Tsror et al. (2008), European Journal of Plant Pathology 123(3): 311-320; S 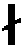 awiak *et al.,* (2009), European Journal of Plant Pathology 125(2): 245-261). This new clade probably constitutes a new species and is provisionally called *"D. solani*". Its occurrence was reported in potato in many European countries (i. e. The Netherlands, Finland, Poland, Germany, Belgium, France, United Kingdom, Sweden and Spain) and in Israel.

Possibilities to control *Dickeya* species and *Pectobacterium* species in potato are limited (see Van der Wolf and De Boer (2007), In: Potato biology and biotechnology, advances and perspectives. pp.595-617, Elsevier, Amsterdam). Control strategies include the use of pathogen-free seed, measures to avoid wounding, drainage of soils to avoid oxygen depletion of tubers which can impair the host resistance, and hygienic measures. These methods, however, do not guarantee production of blackleg pathogen-free crop.

No tuber treatments are currently used in practice to reduce the inoculum of *Dickeya* and *Pectobacterium* species. In general, use of physical treatments and chemical control agents only will help to reduce inoculum superficially present in tubers, but cannot eliminate inoculum deeper located without affecting tuber sprouting. No systemic bactericides are available that can kill the pathogens inside.

Application of micro-organisms as a biological control agent is often an environmental-friendly alternative to traditional physical and chemical treatments.

Although promising control may be obtained for some bacterial diseases in plants like crown gall caused by *Agrobacterium tumefaciens* (see Lopez et al. (1987), EPPO Bulletin 17: 273-279) and fire blight caused by *Erwinia amylovora* (see Stockwell et al. (1998), Phytopathology 88: 506-513) with the use of bacterial antagonists, only limited work has been conducted to control pectinolytic bacteria in potato plants under field conditions. No commercial products against blackleg and soft rot diseases based on bio-control agents or their mixtures have been introduced to the market.

EP 1241247 A1 (C. C. H. S. A.) describes a number of bacterial isolates of different genera. Particularly described are the effects of antagonistic bacteria (i) on *Fusarium* infection of cucumber seeds; (ii) *Fusarium oxysporum* infection of melon seedlings, (iii *Corynebacterium michiganese* infection in tomato plants; (iv) *Botrytis* infection of germinating cucumber seeds; (v) grey rot in tomato caused by *Botrytis cinerea*; and (vi) powdery mildew in tomato seeds caused by *Erysiphae* spp.

*Serratia* is a genus of Gram-negative, facultatively anaerobic, rod-shaped bacteria of the *Enterobacteriaceae* family. The most common species is *Serratia marcescens.* Members of the genus frequently produce prodigiosin which is a characteristic red pigment of the genus.

Antagonistic activity to bacterial plant pathogens exhibited by *Serratia marcescens* has been disclosed earlier. Jafra S. et al (2009, Journal of Applied Microbiology 106: 268 - 277) describes a strain of *Serratia marcescens* able to inhibit maceration caused by *Dickeya* zeae which is a causative agent of soft rot in the bulbs.

Strains of another *Serratia* species, namely *S. plymuthica* have been frequently used to control fungal pathogens of plants (see Berg, G. (2000) Journal of Applied Microbiology 88: 952-960; see also Frankowski et al. (2001) Archives of Microbiology 176: 421-426). A comprehensive review on the use of *Serratia plymuthica* for the control of fungal plant pathogens is provided by De Vleesschauwer and Höfte (2007, CAB Reviews: Perspectives in Agriculture, Veterinary Science, Nutrition and Natural Sources 2: 1-12).

WO 01/40442A1 describes the use of a *Serratia plymuthica* strain and metabolites thereof for the suppression of weeds and fungi. The strain is A153 deposited at NCIMB under accession No. 40938 and its active metabolites are haterumalide A, B, E and X.

KR 2007 0080775 A (Nat Univ Gyeongsang) describes *S*. *plymuthica* strain A21-4 (KACC 91013) which was isolated from *Gyeongsangnam jinseongmyeon* onion roots. The A21-4 strain was selected for control activity against blight of peppers, cucumbers and tomatoes caused by *Phytophthora* spp., *Pythium* spp. *Sclerotium* spp. and *Botrytiscineria* spp.

In spite of the frequent use of *Serratia plymuthica* as BCA against fungal plant pathogens, no substantial antagonistic activity against bacterial plant diseases have been reported so far for this *Serratia* species. For example, a *Serratia plymuthica* strain commercially available for the biocontrol of fungal diseases showed negligible activity to *Dickeya* species causing soft rot and blackleg disease in potato. Of the many strains of *Serratia plymuthica* described so far as potential antimicrobial BCAs, none of these have been reported to show any activity against plant pathogenic bacteria (De Vleesschauwer and Höfte (2007), CAB Reviews: Perspectives in Agriculture, Veterinary Science, Nutrition and Natural Sources 2: 1-12).

The inventors have discovered a new strain of *S. plymuthica* designated A30. The inventors have also found that *S. plymuthica* A30 is a BCA. In particular, that *S. plymuthica* A30 is a BCA against the bacteria causing blackleg disease in potato plants. The newly discovered *S. plymuthica* A30 has been found to be a BCA against pectinolytic *Dickeya* spp., including the biovar 3 strain.

The inventors have found that tuber treatments with *S. plymuthica* A30 as a BCA results in protection of potato plants from blackleg and eradication of tuber-borne *Dickeya* sp. inoculum. *S. plymuthica* A30 has also been found to survive in soil and colonize potato tubers, roots and stems from soil-borne inoculum. The colonizing *S. plymuthica* A30 is also found to be sustained inside potato plants.

Surprisingly it has been found that the presence of *S. plymuthica* A30 decreases blackleg incidence in potato plants by 100% under greenhouse conditions. Also, the presence of *S. plymuthica* A30 decreases the colonization of stems by *Dickeya* sp. by 97% under greenhouse conditions.

Accordingly, the present invention provides *Serratia plymuthica* strain A30, BCCM Deposit No. LMG P-26170 (deposited 19^{th} November 2010).

The depositor of BCCM LMG P-26170 is Wageningen University & Research Centre, Plant Research International, P.O. Box 69, 6700 AB Wageningen, The Netherlands. The authorised representing person is Dr Jan M van der Wolf of the same address. The depositor has consented and authorises the applicant, Stichting Dienst Landbouwkundig Onderzoek and Stichting voor de Technische Wetenschappen to refer to the deposited biological material in the present application.

The strain produces antibiotics against *Dickeya* and *Pectobacterium* species, produces auxin with and without supplementation of L-tryptophan, produces biosurfactants, is motile, is not pectinolytic, grows at pH 10.0 in nutrient broth and under anaerobic conditions in potato dextrose broth. A30 strain does not produce red pigment.

*S. plymuthica* strain A30 is preferably in an isolated form, although it may be present in combination with other bacteria of the same or a different genus, whether the bacteria are brought together by hand of man and co-cultured, or whether bacteria existing together in nature are partially purified from other bacteria with which they exist in nature.

In preferred embodiments, the A30 strain is a substantially pure culture, which means a culture of a bacterial strain containing no other bacterial species in quantities sufficient to interfere with replication of the culture or to be detectable by normal bacteriological techniques.

"Isolated" when used in connection with the organisms and cultures described herein not only means a substantially pure culture, but also any culture of organisms which is grown or maintained other than as it is found in nature.

The *S. plymuthica* strain A30 of the invention may be in a form which is metabolically active, which is to say that it forms a growing culture in liquid and/or on solid medium. Metabolic activity may be determined simply by measuring the growth of bacterial cells or may be determined by measuring the integrity of the cell membrane or the activity of certain enzymes such as DNase, esterase, lipase and/or gelatinase, for example. The activity of other core metabolic bacterial enzymes may be measured to assess metabolic activity.

Alternatively, the *S. plymuthica* strain A30 of the invention may be in a solid form, e.g. or a dried or freeze dried preparation of a culture. In this form the bacterial cells are believed to be substantially inactive metabolically, but on dissolving the dried preparations into an aqueous environment bacterial cell activity is resumed, together with measurable bacterial cell growth and metabolism.

*S. plymuthica* is classified into risk group 1 according to DSMZ (German collection of Microorganisms and Cell cultures), meaning that the species is not expected to pose a risk for humans and environment. To date, no human or animal-related pathogenicity factors for *S. plymuthica* have been described.

The invention also provides a biological culture comprising *Serratia plymuthica* strain A30, Deposit No. LMG P-26170 and a solid or a liquid medium, or a fraction thereof.

The biological culture of the invention may be substantially uncontaminated with other organisms so that it is comprised substantially of *S. plymuthica* A30. The culture may be substantially homogeneous in terms of bacterial cell content, i.e. at least 95% *S. plymuthica* A30; preferably the homogeneity is selected from one of at least: 96%, 97%, 98%, 99%, 99.5% or 99.9%. The biological culture may comprise no other competent or viable bacterial cell other than *S. plymuthica* A30.

The present invention therefore also provides a substantially isolated *S.plymuthica* A30.

The invention further provides a composition for biological control of plant disease comprising *Serratia plymuthica* strain A30 Deposit No. LMG P-26170 and an agriculturally or horticulturally acceptable diluent, carrier, filler or adjuvant.

Compositions within the present invention may be formulated as: aqueous suspensions; stabilized liquid suspensions; emulsifiable concentrates ; capsules; soluble or wettable powders; aqueous flowables; dry flowables; wettable granules; wettable dispersible granules; and the like, as is known to those skilled in the art.

Examples of agriculturally acceptable or horticulturally acceptable diluents include aqeuous solutions of monosaccharides, polysaccharides, molasses, gums, lignosulfonates, glycerol, sorbitol, propylene glycol, and water, vegetable oils and mineral oils. Carriers may include solids such as alginate beads, durum flour (starch) granules, silica, clays, clay minerals (e.g. attapulgite, kaolonite, montmorillonite, pyrophillite, illite), gelatine, cellulose, cellulose derivatives, calcium chlorite and talcum powder. In some embodiments the carrier may be a porous solid, e.g. diatomaceous earth, charcoal, (e.g. animal bone charcoal), peat, vermiculite, lignite, wood chips and corn cob.

The A30 strain may also be incorporated into fertilizers, soil, or foliar additives. Other suitable formulations will be readily apparent to those skilled in the art.

The composition preferably also includes the appropriate amount or concentration of *S. plymuthica* A30, depending on the route and timing of treatment of plants, plant tissue or plant parts against disease. Preferably the biocontrol agent is applied in higher than pathogen inoculum.

The compositions of strain A30 may be applied manually to plants by means of machines (sprayers) or irrigation systems. Plant parts such as tubers may be dipped into aqueous or liquid suspensions or solid powders or sprayed. Several applications of compositions of the invention may be desirable during a crop cycle given the upredictability of the onset of disease caused by pathogenic plant bacteria. Inoculation of plants with strain A30 of the invention is preferred prior to pathogen exposure, whenever possible.

An application protocol in accordance with the invention consists of dipping potato tubers prior to planting in an aqueous suspension of strain A30. The strain A30 is at a concentration in the range 10⁶ to 10¹² colony forming units (cfu) per milliliter (cfu.ml⁻¹). A preferred concentration range for the A30 strain is 10⁸ to 10¹⁰ cfu.ml⁻¹.

Organic and/or inorganic fertilizers can be added with A30 to help build populations of A30 in the foliage, roots, and soil. The fertilizer can be added at the same time or before or after the A30 strain is present. The fertilizer can be added to the plant and/or to the soil.

In preferred embodiments of the invention for use in field conditions, a concentration between 10⁸ to 10¹⁰ cfu.ml⁻¹ of strain A30 is employed. In more preferred embodiments the concentration is in the range 0.5 to 9.5 x 10⁹ cfu. ml⁻¹, In the field, tubers may be treated at the time of planting with as little as about 1 ml of the suspension.

In some embodiments tubers may be dipped in a liquid prior to planting. In preferred embodiments, tubers are sprayed with liquid composition comprising strain A30 of the invention at the time of planting. The spraying may be carried out in ways known in connection with fungicides such as for monceren. In preferred embodiments, the *S. plymuthica* will colonize plants directly upon sprouting and root formation.

Compositions of the invention may further comprise one or more further components which can be a biological control agent, an antibiotic, a herbicide, a pesticide, a fungicide, a plant growth substance an inducer of natural plant defense or a fertilizer. The one or more further components may be administered to plants or plant parts simultaneously, separately or sequentially. The *S. plymuthica* A30 may be administered before or after the other further components.

Of course, the application of further substances alongside strain A30 of the invention, e.g. pesticides, should generally be avoided due to their potential antibacterial activity (unless previously tested in a bioassay). If a pesticide is to be used, then the A30 strain of the invention is preferably reapplied one week after pesticide application.

The invention therefore includes a kit of parts comprising a first container containing the composition of *S. plymuthica* A30, and a second container containing an agriculturally or horticulturally acceptable diluent, carrier or adjuvant. The second container may also comprise the further component such as a biological control agent, an antibiotic, a herbicide, a pesticide, a fungicide, a plant growth substance or a fertilizer.

Alternatively, the second container contains an agriculturally or horticulturally acceptable diluent, carrier or adjuvant, and a yet further container contains the further component such as a biological control agent, an antibiotic, a herbicide, a pesticide, a fungicide, a plant growth substance or a fertilizer.

The kits of the invention may also include written instructions in printed or electronic form.

Other biological control agents may include microorganisms such as fungi, e.g. *Trichoderma harzianum, Trichoderma polysporum* and *Gliacladium virens*, and/or bacteria, e.g. *Agrobacterium radiobacter, Pseudomonas fluorescens* and *Bacillus subtilis* and or bacteriophages

Antibiotics as additional composition components may include streptomycin or actinomycin, for example.

Herbicides as additional composition components may include dimethenamid, EPTC, glyphosphate, paraquat, pendimethalin, sethoxydim, rimsulfuron, metolachlor or metrubuzin.

Pesticides as additional composition components may include 2,4-dichlorophenoxyacetic acid (2,4-D), chloropropham, DDT, DDE, dieldrin, endosulfans, thiabendazole, c-phenylphenol and phorate.

Fungicides as additional composition components may include metalaxyl and/or carbamate compounds, cymoxanil and mancozeb. In particular the synergistic combination of cymoxanil and mancozeb is useful.

Plant growth substances as additional components of the compositions may include an auxin or abscissic acid (ABA). The auxin may be one or more of 2,4-Dichlorophenoxyacetic acid (2,4-D), α-Naphthalene acetic acid (α-NAA), 2-Methoxy-3,6-dichlorobenzoic acid (dicamba), 4-Amino-3,5,6-trichloropicolinic acid (tordon or picloram)or α-(p-Chlorophenoxy)isobutyric acid (PCIB, an antiauxin).

The composition of the invention may further comprise a rooting substrate, e.g. a compost, soil (natural or artificial), sand, and/or an inert particulate rooting medium, such as vermiculite.

Plants, plant tissue or plant parts treated with compositions of the invention acquire an exogenous and/or endogenous infection of *S. plymuthica* A30. Only a single treatment may be necessary, although two or more treatments may be required to achieve optimal infection. Although not wishing to be bound by any particular theory, the inventors believe that the endogenous and/or exogenous infection with *S. plymuthica* A30 results in the prevention of growth of any other bacterial species which may also be present, particularly *Pectobacterium* spp. and/or *Dickeya* spp. The inventors have found that strain A30 is an endophyte which is a microbe which colonizes living, internal tissues of plants without causing any immediate, overt negative effects.

In situations where other bacterial species have already infected a plant, plant part or tissue, treatment with a composition of the invention allows an infecting *S. plymuthica* A30 to eventually outcompete the original bacterial species. Hence the compositions of the invention may be used for prevention or treatment of disease, e.g. soft rot or blackleg in plants.

The invention therefore also includes a plant, plant part or plant tissue comprising exogenous and/or endogenous *Serratia plymuthica* strain A30, Deposit No. LMG P-26170. The range of such plants in accordance with this aspect of the invention is listed below as plants susceptible for treatment in accordance with the methods of the invention.

The invention further includes a method of preventing or treating disease in plants comprising exposing a plant, plant part or plant tissue to *Serratia plymuthica* strain A30, Deposit No. LMG P-26170. Similarly, such methods of the invention include applying compositions of the invention described herein to plants, plant parts or tissue to provide a biological control of infection by plant pathogenic bacteria. Methods of application of the A30 strain and its compositions are well known to the average skilled person, as well as apparatus for mixing and applying compositions to plants, plant parts and plant organs. Subject of these methods may be seeds, seedlings, plants, crops, plant parts, flowers, fruits, vegetative plant parts (e.g. seed tubers and plant cuttings), soil and artificial substrate systems used for culturing plant material. The applications of strain the biological control agent of strain A30 and its compositions can be pre-harvest or post-harvest.

Where plants possess tubers, bulbs, corms or rhizomes, and plants are to be treated in accordance with the invention, then application of compositions of the invention preferably takes place prior to the planting of a tuber, bulb, corm or rhizome. Seeds can also be treated in accordance with the invention prior to sowing. The treatment may take the form of a coating of the seed in which the A30 strain of the invention is incorporated into the a known seed coating mixture. The coated seeds may take the form of pellets.

Alternatively or in addition, whether or not plants possess tubers, bulbs, corms or rhizomes, compositions of the invention may be applied to a shoot or shoot portion of a plant.

The invention therefore includes *S. plymuthica* strain A30, Deposit No. LMG P-26170, as a biological control agent for use in prevention or treatment of a plant disease.

The methods and uses of the invention are preferably for preventing or treating a plant disease is caused by a bacterial disease pathogen, in particular a soft rot or "blackleg" of potato. The bacterial pathogen to be treated or prevented is preferably *Dickeya* spp. and/or *Pectobacterium* spp., preferably *Dickeya* spp. biovar 3 strain.

A wide range of plants are susceptible for prevention or treatment of bacterial disease by using the methods and composition of the invention described herein. Plants may be monocots or dicots.

Amongst monocots susceptible to infection by *Dickeya* spp., the plants may be a species member of the *Araceae, Arecaceae, Asphodelaceae, Bromeliaceae, Hyacinthaceae, Iridaceae, Musaceae, Orchidaceae, Poaceae,* or the *Zingiberaceae.*

The plant susceptible to infection by *Dickeya* spp. may be a species from monocot genera selected from *Aglaonema* spp., *Aechema* spp., *Dieffenbachia* spp., *Philodendron* spp., *Syngonium* spp., *Xanthosoma* spp., *Zantedeschia* spp., *Phoenix* spp., *Aloe* spp., *Ananas* spp., *Iris* spp., *Musa* spp., *Phalaenopsis* spp., *Brachiaria* spp., *Oryza* spp., *Saccharum* spp, *Sorghum* spp., *Zea* spp., or *Elettaria* spp. Preferred species include *Aglaonema pictum, Aechema fasciata, Dieffenbachia* spp., *Philodendron selloum, Syngonium podophyllum, Xanthosoma sagittifolia*, *Zantedeschia aethopica*, *Phoenix dactylifera*, *Aloe vera, Ananas comosus, Iris x germanica*, *Musa paradisiaca*, *Phalaenopsis* spp., *Brachiaria* spp., *Oryza sativa, Saccharum officinarum*, *Sorghum bicolor,* Zea *mays,* or *Elettaria cardomomum.*

Amongst dicots, the plant susceptible to infection by *Dickeya* spp. may be a species member of the *Apiaceae, Asteraceae, Begoniaceae, Brassicaceae, Caryophyllaceae, Convolvulaceae, Crassulaceae, Euphorbiaceae, Fabaceae, Geraniaceae, Gesneriaceae, Myrsinaceae* or the *Solanaceae.*

The plant susceptible to infection by *Dickeya* spp. may be a species from dicot genera selected from *Arracacia* spp., *Daucus* spp., *Chrysanthemum* spp., *Cichorium* spp., *Cynara* spp., *Dahlia* spp., *Begonia* spp., *Dianthus* spp., *Ipomoea* spp., *Kalanchoe* spp., *Euphorbia* spp., *Helianthus* spp., *Medicago* spp., *Parthenium* spp., *Pelargonium* spp., *Saintpaulia* spp., *Cyclamen* spp., *Solanum* spp. or *Nicotiania* spp. Preferred species include *Arracacia xanthorrhiza, Daucus carota, Chrysthanthemum maximum, Chrysanthemum x morifolium, Cichorium intybus, Cynara cardunculus, Dahlia sp., Begonia bertinii, Dianthus caryophyllus, Ipomoea batatus, Kalanchoe blossfeldiana, Euphorbia pulcherrima,Helianthus annuus, Medicago sativa, Pelargonium capitatum, Saintpaulia ionantha, Cyclamen sp., Solanum lycopersicum, Nicotiana tabacum, Solanum melongena* and *Solanum tuberosum.*

Amongst monocots susceptible to infection by *Pectobacterium* spp., the plants may be a species member of the *Agavaceae, Alliaceae, Araceae, Asphodelaceae, Bromeliaceae, Dioscoreaceae, Iridaceae, Lilaceae, Orchidaceae, Pandanaceae, Ruscaceae or Strelitzaceae.*

The plant susceptible to infection by *Pectobacterium* spp. may be a species from monocot genera selected from Agave spp., *Allium* spp., *Dieffenbachia* spp., *Scindapsus* spp., *Zantedeschia* spp., *Aloe* spp., *Dioscorea* spp., *Iris* spp., *Tulipa* spp., *Cattleya* spp., *Cymbidium* spp., *Phalaenopsis* spp., *Pandanus* spp., *Dracaena* spp, Strelitzia spp. Preferred species include *Agave tequilana, Allium cepa, Dieffenbachia* spp., *Scindapsus aureus, Zantedeschia aethiopica, Zantedeschia elliottiana, Zantedeschia rehmannii, Aloe arborescens, Dioscorea* spp., *Tulipa* spp., *Cattleya* spp., *Cymbidium* spp., *Phalaenopsis* spp., *Pandanus conoideus, Dracaena sanderiana, Strelitzia reginae.*

Amongst dicots, the plant susceptible to infection by *Pectobacterium* spp. may be a species member of the *Amaranthaceae, Anacardiaceae, Apiaceae, Asteraceae, Brassicaceae, Cactaceae, Crassulaceae, Cucurbitaceae, Euphorbiaceae, Lauraceae, Malvaceae, Moraceae, Myrsinaceae, Primulaceae,* Rosaceae or the *Solanaceae.*

The plant susceptible to infection by *Pectobacterium* spp. may be a species from dicot genera selected from *Beta* spp., *Spinacea* spp., *Mangifera* spp., *Apium* spp., *Arracacia* spp., *Coriandrum* spp., *Daucus* sp., *Arctium* spp. *Cichorium* spp., *Helianthus* spp., *Lactuca* spp., *Parthenium* spp., Tagetes spp., *Eutrema* spp., *Brassica* spp., *Raphanus* spp., *Acanthocereus* spp., *Carnegiea* spp., *Ferocactus* spp., *Opuntia* spp., *Stenocereus* spp., *Kalanchoe* spp. Preferred species include *Beta vulgaris, Spinacea oleracea, Mangifera indica, Apium graveolens, Arracacia xanthorrhiza, Coriandrum sativum, Daucus carota, Arctium minus, Cichorium intybus, Helianthus annuus, Lactuca sativa, Parthenium argentatum, Tagetes patula, Eutrema wasabi, Brassica oleracea, Brassica rapa, Brassica sativus, Raphanus sativus, Acanthocereus tetragonus, Carnegiea giganteum, Ferocactus wislizenii, Opuntia ficus-indica, Opuntia fulgida, Opuntia phoeacantha, Opuntia stricta, Opuntia violacea, Stenocereus thurberi, Kalanchoe blossfedliana.*

Advantageously, the invention provides a biological control agent of low toxicological impact on the environment, low risk for resistance development by the pathogens. There is also the relatively durability/longevity of the biocontrol effect after settlement of the A30 strain in the plant system.

The efficacy of strain A30 is relatively broad; for example, in contrast to an antagonistic *Bacillus* species the A30 strain on potato tuber disks has strong antagonistic activity against both *Dickeya* and *Pectobacterium* species.

Another advantage of the A30 strain is that it is an exceptionally potent internal and external colonizer of plant roots and this contributes to its success as a biological control agent. These advantages of strain A30 were entirely unexpected, particularly as a combination.

The invention will now be described in detail and with reference to the Examples and to the following drawings in which:

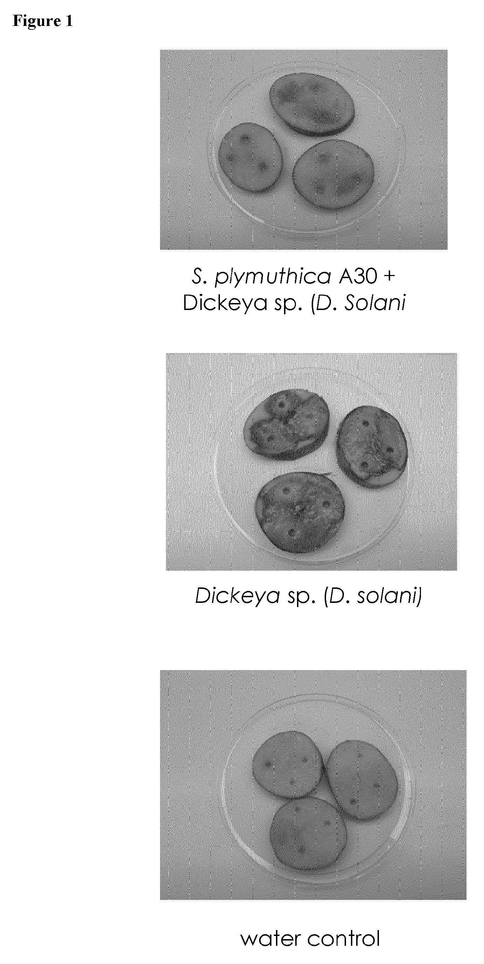

**Figure 1****.** shows the protective effect of applied *S. plymuthica* A30 on potato slices against maceration caused by biovar 3 *Dickeya* sp. The ability of a GFP-tagged strain of *S. plymuthica* A30 to protect potato tuber tissue against maceration by *Dickeya* sp. was evaluated in a potato slice assay. A30 and *Dickeya* sp. IP02222 were grown overnight in NB at 28 °C with shaking (200 rpm). Bacteria were centrifuged (5 min, 6000 x g) and washed twice with 1/4 Ringer's buffer. The density of A30 was adjusted to approx.10⁸ cfu ml⁻¹ and *Dickeya* sp. to approx.10⁶ cfu ml⁻¹ with sterile water. Wells of the tuber were filled up with 50 µl suspension containing 10⁸ cfu ml⁻¹ of A30 and 10⁶ cfu ml⁻¹ of *Dickeya* sp. Three potato slices derived from three different tubers were used per treatment. For the negative control, instead of bacterial suspension 50 µl of sterile water and for the positive control 50 µl containing 10⁶ cfu ml⁻¹ of *Dickeya* sp. were used. The experiment was independently repeated. For disease development slices were incubated at 28 °C for 72 h in a humid box. The protection effect of A30 on potato tissue was measured by comparing the average diameter of rotten potato tissue around co-inoculated wells with the average diameter of rotten potato tissue around wells of the positive control.

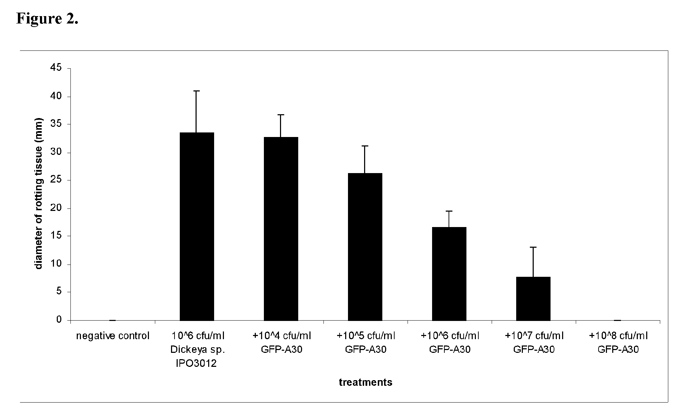

**Figure 2****.** attenuation of maceration ability of *Dickeya* sp. IPO3012 by application of *S. plymuthica* A30 determined by measuring the diameter of rotting tissue (in mm) after 72 h incubation at 28 °C in humid box. Wells of potato slices were inoculated with 50 µl of sterile water (negative control) with 50 µl bacterial suspension in water containing 10⁶ cfu ml⁻¹ of *Dickeya* sp. IPO3012 (positive control) or with 50 µl of bacterial suspension in water containing 10⁶ cfu ml⁻¹ of *Dickeya* sp. IPO3012 and different densities of *S. plymuthica* A30 (0, 10⁴, 10⁵, 10⁶, 10⁷ and 10⁸ cfu ml⁻¹). Three potato slices containing 3 wells each and derived from three different tubers were used per treatment. The experiment was independently repeated one time and the results were averaged.

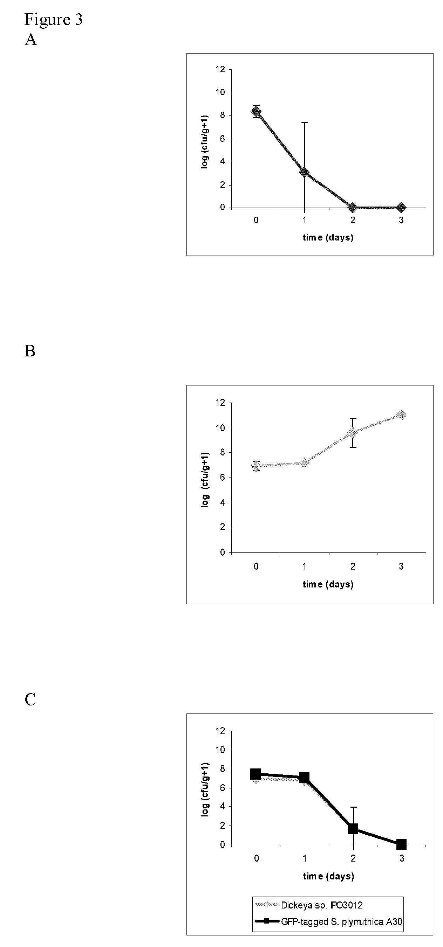

**Figure 3****.** shows the population dynamics of GFP-tagged *S. plymuthica* A30 and *Dickeya* sp. IPO3012 on potato slices. Potato slices were inoculated with GFP-tagged *S. plymuthica* A30 (Figure 3A), DsRed-tagged *Dickeya* sp. IPO3012 (Figure 3B) or co-inoculated with both strains (Figure 3C). Bars represent standard error.
   To assess bacterial densities on potato slices, approx 2 g of tuber tissue independently collected from 3 randomly chosen wells per treatment and per time point was crushed in the presence of 4 ml of 1/4 Ringer's buffer in Universal Bioreba bag using a hammer. Hundred µl of undiluted, 1000 and 10,000 times diluted extracts were NA pour plated.
   The medium was supplemented with 40 µg ml⁻¹ of tetracycline. Plates were incubated at 28 °C for 24-48 h and screened using epifluorescence stereo microscope for presence of DsRed and/or GFP fluorescent cells. Results from free independent samples per treatment and per time point were averaged. The experiment was independently repeated.

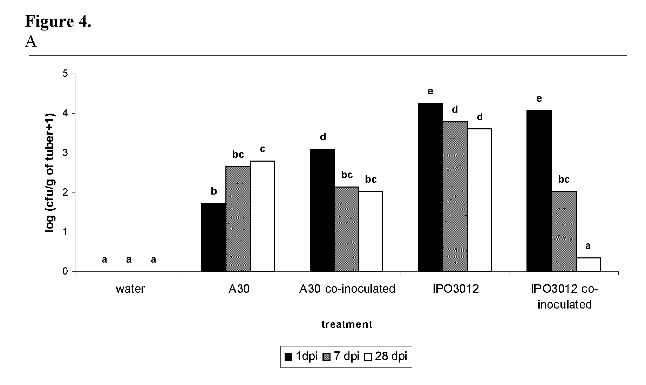

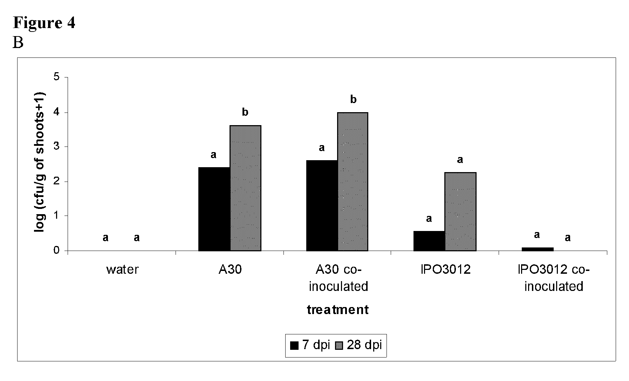

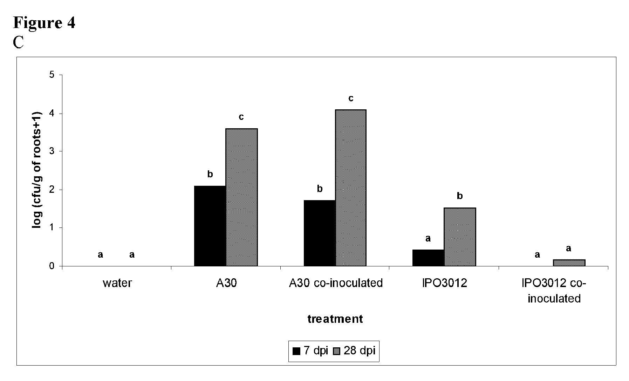

**Figure 4**. shows the population dynamics of GFP-tagged *S. plymuthica* A30 and DsRed-tagged *Dickeya* sp. IPO3012 in seed tubers (Figure 4A), shoots (Figure 4B), and roots (Figure 4C) sampled at 1, 7 and 28 days after inoculation. Plant samples were surface-sterilized before extraction.
   Entire seed tuber and total roots were sampled per plant at each time point. At 7 days post inoculation, all shoots were sampled as a composite sample per plant and at 28 days composite samples of stem cuttings taken 5 cm above the ground level was per plant analyzed. Plant samples were surface-sterilized before extraction of bacteria. Plant extracts were NA pour plated and after incubation plates were screened for green and/or red fluorescent colonies. The average values from two experiments are shown from ten plants per time point. Statistical analysis was done per subsample and per time point (soil n=10, total roots n=10, stems n=10, seed tubers n = 10). Values followed by identical characters are not significantly different (P=0.05)

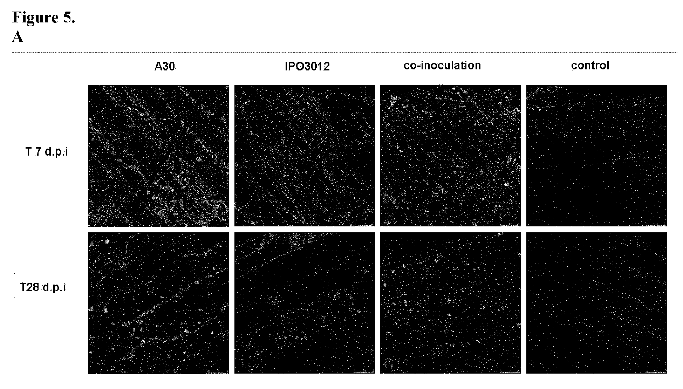

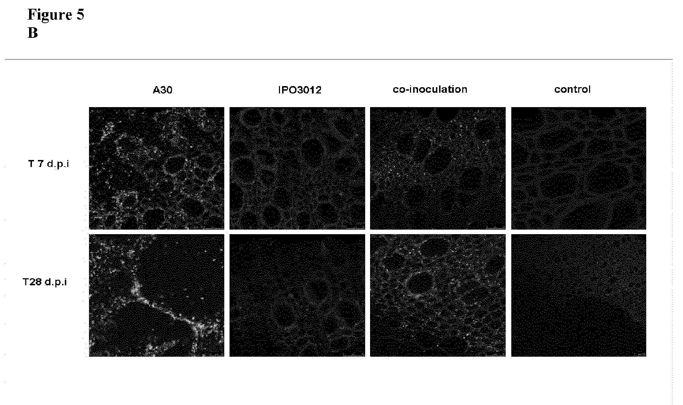

**Figure 5****.** shows the internal colonization of potato roots (Figure 5A) and stems (Figure 5B) by GFP-tagged *S. plymuthica* A30 (green) and DsRed-tagged *Dickeya* sp. IPO3012 (red) at 7 and 28 days post inoculation, analyzed by confocal laser scanning microscopy after incubation of embedded plant parts in NA agar for 1-2 days at 28 °C to allow bacteria to grow. Samples were taken from plants for which tubers were inoculated with GFP-tagged *S. plymuthica* A30 (A30), from plants raised from tubers vacuum infiltrated with DsRed-tagged *Dickeya* sp. (*IPO3012*) and after sequential-inoculation of tubers with both strains *(co-inoculation).* For control, potato tubers were vacuum infiltrated with sterile water *(water).* For counter staining of plant cells, UV light was used.

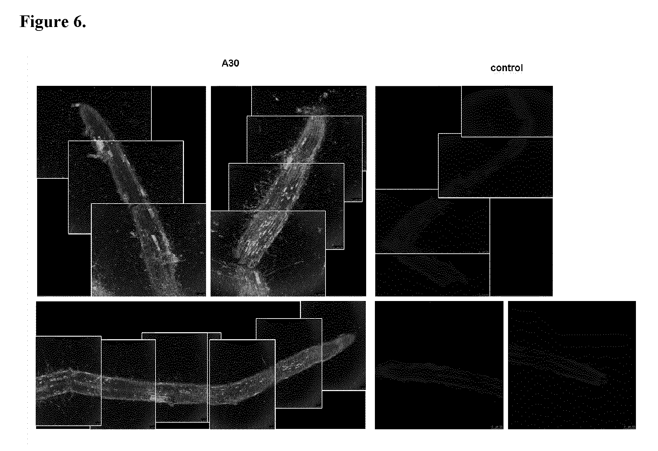

**Figure 6** shows colonization of the surface of freshly collected potato roots by GFP-tagged S. *plymuthica* A30 (green clumps), 28 days post soil infestation. Roots were analyzed with a confocal laser scanning microscopy on freshly collected plant samples. For this roots were collected, briefly washed in sterile tap water to remove soil particles and directly processed for microscopy. For control, roots of water inoculated plants were prepared and processed in the same way. For counter staining of plant cells, UV light was used.

In greenhouse experiments carried out by the inventors, treatment of tubers with GFP-tagged *S. plymuthica* A30 just before planting resulted in protection of potato tubers/plants from *Dickeya* sp. and blackleg symptoms. Percentages of plants expressing tuber rot or typical blackleg symptoms were significantly reduced in comparison to the control plants inoculated with *Dickeya* sp. IPO3012.

Tuber treatments with GFP-tagged *S. plymuthica* A30 resulted in a rapid colonization of potato plants. Potato plants were systematically and internally colonized just 7 days post bacteria application to soil. *S. plymuthica* A30 was able to move inside potato plants upward to the stems after inoculation of soil. The bacteria then survived in the potato tissues. Movement of bacteria occurred via xylem vessels in vascular tissue of roots and stems. Relatively low yet stable populations of *S. plymuthica* A30 inside roots and stems were present for at least 28 days. *S. plymuthica* A30 shares the characteristic of typical endophyte which does not cause any disease symptoms in plants and build up only low internal populations. These low A30 populations inside roots and stems were sufficient to protect potato plants from colonization by *Dickeya* sp. IPO3012 and expression of typical blackleg symptoms in majority of co-inoculated plants.

The inventors also found that *S. plymuthica* A30 was also able to colonize the root surface. After 7 days bacteria were already present inside vascular tissue of roots and after 28 days we observed large A30 populations on the root surface.

The inventors further observed that *S. plymuthica* A30 was able to efficiently colonize internal root tissues suggesting that the bacterium is able to invade roots via natural openings created during root development.

### EXAMPLE 1 - Characterization of bacteria antagonistic to Dickeya solani

Bacteria were isolated from rotten potato tuber tissue of different tubers (cv. Arcade, Konsul, Kondor, Agria) by plating rotten potato tuber extracts on agar media TSA, NB, King's B or R2A and collecting morphologically different bacterial colonies. 649 isolated strains were screened for antibiosis against *D. solani* or for the production of iron ion chelating proteins (siderophores). Of these, 112 isolates produced siderophores, 41 produced antibiotics and 496 were not active against *D. solani.*

A selection of 41 antibiotic-producing strains and 41 siderophore producing strains were tested in a potato slice assay for antagonism against *D. solani.* Strains that were able to reduce rotting of potato tuber tissue to at least 50% of the control were selected. These isolates then characterized by 16S rRNA sequencing as being species of *Bacillus, Pseudomonas, Rhodococcus, Serratia, Obesumbacterium* or *Lysinbascillus.*

23 of these isolates, 13 producing antibiotics and 10 producing siderophores, were further characterised by testing quorum sensing signal detection, motility, biosurfactant production, growth at low (4.0) and high (10.0) pH, growth at 10 °C in aerobic and anaerobic conditions and for tryptophan dependent and independent auxin production.

12 of the above isolates were taken and tested in plants in the greenhouse. Potato tubers were inoculated with each isolate.

As a result of the greenhouse experiments, four antagonistic strains were found.

One of these is *Serratia plymuthica* A30 which was deposited under the Budapest Treaty at the Belgian Co-ordinated collection of Microorganisms (BCCM) under accession No. LMG P-26170.

The strain was selected on the basis of *in vitro* production of antibiotics against *Dickeya* sp. The A30 strain produces antibiotics against *Dickeya* sp., is able to degrade acyl homoserine lactones and produces biosurfactants. The strain is motile and able to grow in aerobic and anaerobic conditions. The A30 strain also produces plant growth stimulating auxins.

### Example 2 - Analysis of the ability of S. plYmuthica A30 to protect potato tuber tissue against maceration by Dickeya sp. IPo2222

The ability of *S. plymuthica* A30 to protect potato tuber tissue against maceration by *Dickeya* sp. IPO2222 was evaluated in a potato slice assay. A30 and *Dickeya* sp. IPO2222 were grown overnight in NB at 28 °C with shaking (200 rpm). Bacteria were centrifuged (5 min, 6000 x g) and washed twice with 1/4 Ringer's buffer. The density of A30 was adjusted to approx.10⁸ cfu ml⁻¹ and *Dickeya* sp. to approx. 10⁶ cfu ml⁻¹ with sterile water. Wells of the tuber were filled up with 50 µl suspension containing 10⁸ cfu ml⁻¹ of A30 and 10⁶ cfu ml⁻¹ of *Dickeya* sp. Three potato slices derived from three different tubers were used per treatment. For the negative control, instead of bacterial suspension 50 µl of sterile water and for the positive control 50 µl containing 10⁶ cfu ml⁻¹ of *Dickeya* sp. were used. The experiment was independently repeated. For disease development slices were incubated at 28 °C for 72 h in a humid box. The protection effect of A30 on potato tissue was measured by comparing the average diameter of rotten potato tissue around co-inoculated wells with the average diameter of rotten potato tissue around wells of the positive control. The results showed a clear and strong protective effect of S. plymuthica A30 against maceration by *Dickeya* sp. IPO2222 (see figure 1).

### EXAMPLE 3 - Construction of marker strains tagged with GFP or RFP proteins

To assess knowledge of interaction and bacterial populations inside potato plant tissue and to microscopically visualize viable bacterial cells *in situ*, including *in planta,* the biovar 3 *Dickeya* sp. strain was tagged with red fluorescent protein (DsRed) and *S. plymuthica* A30 with green fluorescent protein (GFP), respectively. Tagging with GFP and DsRed proteins enabled detailed microscopy study on the localization and population dynamics of both bacteria species.

### Bacterial strain and media used for cultivation

*S. plymuthica* A30 (Deposit Accession No. LMG P-26170) and biovar 3 type strain *Dickeya* sp. IPO2222 (S 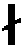 awiak et al. (2009),, European Journal of Plant Pathology 125: 245-261) were grown at 28 °C for 24-48 h on tryptic soya agar (TSA) (Oxoid) or nutrient agar (NA) (Oxoid) prior to use. Liquid cultures were prepared in nutrient broth (NB) (Oxoid) and/or tryptic soya broth (TSB) (Oxoid) at 28 °C for 24 h with agitation (200 rpm). Strains of GFP-tagged *S. plymuthica* A30 and DsRed-tagged *Dickeya* sp. IPO3012 were grown on or in the same media supplemented with 40 µg ml⁻¹ of tetracycline (Sigma) (NAt, TSAt, NBt, TSBt). When plant extracts were analyzed, growth media were additionally supplemented with cycloheximide (Sigma) to a final concentration of 200 µg ml⁻¹ to prevent possible fungal growth.

### Generation of GFP-tagged S. plymuthica A30 and DsRed-tagged Dickeya sp. IPO3012 strains

Plasmids pRZ-T3-*gfp* and pRZ-T3-*dsred* were used for generation of GFP-tagged *S. plymuthica* A30 (A30GFP) and DsRed-tagged biovar 3 *Dickeya* sp. IPO3012 (parental strain *Dickeya* sp. IPO2222), respectively. The plasmids carrying genes coding for fluorescent proteins were introduced to bacterial cells by electroporation (see Calvin and Hanawalt (1988), Journal of Bacteriology 170: 2796-2801) as described previously (see Czajkowski et al. (2010), Phytopathology 100: 134-142). Briefly, suspensions of competent bacterial cells of approximately 50 µl of A30 or IPO2222 (containing approx. 10¹¹-10¹² cfu. ml⁻¹ were mixed with 100 ng µl⁻¹ plasmid DNA and electroshocked at 2.5 kV for 1-2 sec at 4°C using Bio-Rad Gene Pulser 200/0.2 (Biorad, Hercules, CA, USA). After electroporation, bacterial cells were resuscitated for 1 h in 500 µl of NB at 28 °C with shaking. Hundred microliters of the transformed cells were plated on TSA containing 40 µg ml⁻¹ of tetracycline and incubated for 24-48 h at 28 °C for selection of positive GFP or DsRed fluorescent transformants.

Transformation of *S. plymuthica* A30 with pRZ-T3-*gfp* and *Dickeya* sp. IPO2222 with pRZ-T3-*dsred* plasmids resulted in 43 and 29 transformants, respectively. A colony with a high fluorescence was collected for each of the bacteria. Repeated transfers of transformants on agar plates under selective conditions showed that bacteria expressed GFP or DsRed in a stable way. The presence of pRZ-T3-*gfp* in *S. plymuthica* A30-GFP and pRZ-T3-*dsred* in *Dickeya* sp. IPO3012 was proven by plasmid DNA purification and agarose gel electrophoresis.

Labeling of *S. plymuthica* A30 and *Dickeya* sp. IPO2222 with fluorescent proteins did not affect the fitness of the strains. The transformed strains behaved in the same way as the parental wild-type strains indicating that the expression of GFP and DsRed proteins did not significantly affected the important biological features.

### EXAMPLE 4 - Growth of GFP and DsRed tagged bacterial strains compared to growth of parental strains

To compare bacterial growth of tagged bacteria with parental strains *(Dickeya* sp. IPO3012 versus IPO2222 and A30 versus A30GFP) under aerobic conditions, an overnight bacterial culture with a density of ca. 10⁹-10¹⁰ cfu ml⁻¹ in NBt was diluted 50 times in the same medium. Bacteria were grown at 28 °C with a shaking rate of 200 rpm. The growth rate was determined by measuring the OD₆₀₀ for a period of up to 24 h.

Growth of *Dickeya* sp. strains under anaerobic conditions, created by adding 5 ml of liquid paraffin to 30 ml of the bacterial suspensions in PEB (see Perombelon, M., C, M, and J. M. Van der Wolf (2002). Methods for the detection and quantification of Erwinia carotovora subsp. atroseptica (Pectobacterium carotovorum subsp. atrosepticum) on potatoes: a laboratory manual, Scotish Crop Research Institute), was determined in a similar way as described for growth under aerobic conditions with the exception that cultures were not agitated during the incubation time.

*S. plymuthica* A30-GFP and *Dickeya* sp. IPO3012 displayed similar growth characteristics in liquid media as the parental wild type A30 and IPO2222 strains, respectively, indicating that the growth of the strains was not affected either by the presence of the pRZ-T3 plasmids or by expression of fluorescent (GFP or DsRed) proteins.

### EXAMPLE 5 - Tuber tissue maceration capacity of a DsRed-tagged Dickeya sp.

Bacterial suspensions were diluted in Ringer's buffer (Merck) to a concentration of approximately 10⁶ cfu ml⁻¹. Potato tubers of cultivar Agria (Agrico, The Netherlands) were rinsed with running tap water, subsequently washed twice with 70 % ethanol for 5 min and washed twice for 1 min with demineralized water. Tubers were dried with tissue paper and cut into 0.7 cm transverse disk slices.

Three 5 mm deep wells per slice were made using a sterile cork borer with a diameter of 5 mm. Wells were filled with 50 µl of the bacterial suspension. Three potato slices derived from three different tubers were used per treatment. For disease development slices were incubated at 28 °C for 72 h in a humid box. The diameter of rotting tissue around inoculated wells was measured after 72 h incubation at 28 °C. The result was compared with that of the wild type strain and with a water control. The experiment was repeated twice with the same setup.

The abilities of the *Dickeya* sp. IPO3012 and IPO2222 to macerate potato tuber tissue were compared in a potato slice test. After incubation of inoculated tuber slices for 3 days at 28 °C, the diameters of the rotting tissue were not significantly different from the wild type IPO2222 strain.

In this potato slice assay, GFP-tagged *S. plymuthica* A30 was able to fully stop potato tissue maceration caused by *Dickeya* sp. IPO3012 when applied in 100 times higher densities than *Dickeya* sp. and considerably reduce tuber tissue maceration when applied in 10 times higher or equal to *Dickeya* sp. densities.

### EXAMPLE 6 - Ability of S. plymuthica A30-GFP to inhibit growth of Dickeya sp. in an overlay plate assay

The ability of GFP-tagged *S. plymuthica* A30 to inhibit growth of *Dickeya* sp. IPO2222 was tested in an overlay plate assay with IPO2222 as the indicator strain. Fifty µl of an overnight culture of *Dickeya* sp. (approx. 10⁹ cfu ml⁻¹) in NB was mixed with 5 ml of soft top agar (NB supplemented with 0.7% agar) pre-warmed to 45-50 °C, and poured onto TSA plates. After agar had solidified, 2.5 µl of an overnight culture of *S. plymuthica* A30 or GFP-tagged *S. plymuthica* A30 in NB and NBt, respectively (approx. 10⁹ cfu ml⁻¹) was spotted on the surface of the agar plate. Plates were incubated for 24 - 48 h at 28 °C. The diameter of the clear 'halo' (indicating *Dickeya* sp. IPO2222 growth inhibition) which appeared around the colonies was measured.

After incubation of co-inoculated agar plates for 1-2 days at 28 °C, the diameters of the clear halos around the GFP-tagged A30 colonies indicating growth inhibition of *Dickeya* sp. were not significantly different from the diameters of the clear halos around the wild type A30 colonies.

### EXAMPLE 7 - Ability of S. plymuthica A30-GFP to protect tuber tissue from soft rot by Dickeya sp.

The ability of GFP-tagged *S. plymuthica* A30 to protect potato tuber tissue against maceration by *Dickeya* sp. IPO2222 was evaluated in a potato slice assay, in a similar way as described for the experiment in which tuber tissue maceration ability of the DsRed-tagged *Dickeya* sp. was tested. GFP-tagged A30 and wild type A30 and *Dickeya* sp. IPO2222 were grown overnight in NBt or NB respectively at 28 °C with shaking (200 rpm). Bacteria were centrifuged (5 min, 6000 x g) and washed twice with 1/4 Ringer's buffer. The density of the GFP-tagged A30 was adjusted to approx. 10⁸ cfu ml⁻¹ and *Dickeya* sp. to approx. 10⁶ cfu ml⁻¹ with sterile water. Wells of the tuber were filled up with 50 µl suspension containing 10⁸ cfu ml⁻¹ of GFP-tagged A30 and 10⁶ cfu ml⁻¹ of *Dickeya* sp. IPO3012.

Three potato slices derived from three different tubers were used per treatment. For the negative control, instead of bacterial suspension 50 µl of sterile water and for the positive control 50 µl containing 10⁶ cfu ml⁻¹ of *Dickeya* sp. were used. The experiment was independently repeated. For disease development slices were incubated at 28 °C for 72 h in a humid box. The protective effect of A30 on potato tissue was measured by comparing the average diameter of rotten potato tissue around co-inoculated wells with the average diameter of rotten potato tissue around wells of the positive control.

The ability of the GFP-tagged *S. plymuthica* A30 to protect potato tuber tissue from maceration by *Dickeya* sp. IPO2222 was compared with the wild type strain of *S. plymuthica* A30 in a potato slice test. After incubation of tuber slices, co-inoculated with a GFP-tagged *S. plymuthica* A30 and *Dickeya* sp. IPO2222 for 72 h at 28 °C the diameters of the rotten tissue were not significantly different.

### EXAMPLE 8 - Effect of density of S. plymuthica A30-GFP on tuber maceration by Dickeya sp.

The effect of the inoculum density of the GFP-tagged *S. plymuthica* A30 on the ability to protect potato tuber tissue against maceration caused by *Dickeya* sp. IPO3012 was tested in a potato slice assay. Potato slices were co-inoculated with 10⁶ cfu ml ⁻¹ of IPO3012 and A30 in density ranging from 10⁴ to 10⁸ cfu ml ⁻¹. GFP and DsRed tagged colonies were counted under an epifluorescence stereo microscope (Leica Wild M32 FL4) equipped with a mercury high pressure photo-optic lamp (Leica Hg 50W/AC) and a GFP and RFP plus filters.

Figure 2 shows that maceration of potato tissue by *Dickeya* was completely stopped at a minimum density of 10⁸ cfu ml ⁻¹ of A30. At lower densities of 10⁷ cfu ml ⁻¹ and 10⁶ cfu ml⁻¹, A30 significantly reduced potato tissue maceration but still rotting of potato slices was observed. At a density of 10⁴ cfu ml ⁻¹, A30 did not reduce tissue maceration (see Figure 2).

### EXAMPLE 9 - Population dynamics of S. plymuthica A30 and Dickeya sp. on potato tuber slices

The population dynamics of *S. plymuthica* A30-GFP and DsRed-tagged *Dickeya* sp. IPO3012 after (co-)inoculation of potato slices was studied for a period of 3 days using pour plating.

The experiment was similar to that described in Example 7 and 8. Tuber wells were filled with a 50 µl suspension containing 10¹⁰ cfu ml⁻¹ of A30 and 10⁸ cfu ml⁻¹ of *Dickeya* sp. IPO3012. Prepared potato slices were incubated at 28 °C in humid boxes for development of rotting. The experiment was independently repeated. To assess the densities of A30 and IPO3012 on potato slices, approx. 2 g of tuber tissue from 3 randomly chosen wells per treatment and per time point were collected daily and crushed in the presence of 4 ml of 1/4 strength Ringer's buffer in Universal Extraction Bag (BIOREBA) using a hammer. 100 µl of undiluted and 1000 times and 10000 times diluted tuber extracts were mixed with pre-warmed to 48 °C NA supplemented with tetracycline (NAt) to the final concentration of 40 µg ml⁻¹ and poured into the wells of 24-well plate (Greiner). After agar had solidified plates were covered with parafilm and incubated at 28 °C for 24-48 h for growth of bacterial colonies. GFP and DsRed tagged colonies were counted under an epifluorescence stereo microscope (Leica Wild M32 FL4) equipped with a mercury high pressure photo-optic lamp (Leica Hg 50W/AC) and a GFP and RFP plus filters.

Figure 3A shows that during a three day period, the density of GFP-tagged *S. plymuthica* A30 decreased rapidly from 10⁷ - 10⁸ cfu g⁻¹ at 0 days after inoculation to 10¹ - 10² cfu g⁻¹ at 2 days after inoculation and 0 cfu g⁻¹ at 3 days after inoculation. No rotting of potato slices was observed during the course of the experiment, although after 3 days a slight brown discoloration of tuber tissue was found similar as to the water control.

Figure 3B shows that during a three day period, the density of *Dickeya* sp. IPO3012 increased from on average 10⁷ cfu g⁻¹ to 10¹¹ cfu g⁻¹. This strong increase was accompanied by a progressive rot of potato slices.

Figure 3C shows that during a three day period, co-inoculation of potato tuber slices with GFP-tagged A30 strain and PO3012 resulted in a simultaneous decrease in the density of both strains. No GFP or DsRed tagged bacteria were recovered from inoculated potato slices at 3 days after inoculation.

The studies on population dynamics on potato slices co-inoculated with GFP-tagged *S. plymuthica* A30 and DsRed-tagged *Dickeya* sp. IPO3012 showed that the biocontrol agent is able to largely reduce populations of *Dickeya* sp. in potato slices just 2 days post application and completely eradicate *Dickeya* sp. from artificially inoculated tubers after 72 h. Control of biovar 3 *Dickeya* sp. by *S. plymuthica* A30 is the most effective at a close distance, possible by cell-to-cell contact.

### EXAMPLE 10 - Greenhouse experiments using tubers treated with Dickeya and S. plymuthica A30, via a soil treatment

### Inoculation of potato tubers with DsRed-tagged Dickeya sp. IPO3012 and soil with GFP-tagged S. plymuthica A30

Greenhouse experiments were conducted in the months of June - July and September- October. Suspensions of DsRed-tagged *Dickeya* sp. IPO3012 were prepared in sterile demineralized water to achieve densities of 10⁶ cfu ml⁻¹. *Dickeya* spp.-free minitubers of cv. Kondor (Dutch Plant Inspection Service for agricultural seeds and seed potatoes (NAK), Emmeloord, The Netherlands) were used. Minitubers were immersed in the suspension and vacuum infiltrated for 10 min at -800 mBar in an exicator followed by 10 min incubation at atmospheric pressure. Minitubers were infiltrated with sterile demineralized water only, served as negative controls. After inoculation tubers were dried in flow cabinet overnight and the next day they were planted in 5 L plastic pots in a sandy rich potato soil (2.9% of organic mater, 0.2% CaCO₃, pH 6.4) collected from a potato field in Wageningen, The Netherlands.

Suspensions of GFP-tagged *S. plymuthica* A30 were prepared in sterile demineralized water to a density of 10¹⁰-10¹¹ cfu ml⁻¹. Planted tubers were watered with tap water 1 h before inoculation. Negative control tubers or *Dickeya* sp. IPO3012 inoculated tubers were inoculated with 50 ml of 10¹⁰-10¹¹ cfu ml⁻¹ GFP-tagged *S. plymuthica* A30 applied directly to the tuber before burying. Pots were kept dewatered 24 h after treatments. Pots were kept in the greenhouse at a 16/8 h photoperiod, 70 % relative humidity and 28 °C for 4 weeks (28 days) after tuber planting. To eliminate the bias effect of growth conditions, a complete random block design of the pots was applied (3 blocks containing 10 pots for each treatment - 40 pots in total per block). In each experiment, 30 Kondor minitubers were used per treatment. In total 120 plants were used: per replication and per time point we used 10 plants inoculated with *Dickeya* sp. IPO3012 (positive control), 10 plants inoculated with sterile water (negative control), 10 plants co-inoculated with *Dickeya* sp. IPO3012 and GFP-tagged *S. plymuthica* A30, and 10 plants inoculated with GFP-tagged S. *plymuthica* A30.

A suspension of the GFP-tagged *S. plymuthica* A30 was applied on the surface of the tubers previously infiltrated with DsRed-tagged *Dickeya* sp. IPO3012 directly after planting and just before covering tubers with soil to mimic an application at planting in the field. The possibility of the strain to colonize potato plants after tuber treatments was studied, to determine the potential of the antagonist for control of *Dickeya* in internal plant tissues.

In this study, the conditions for the greenhouse experiments were adjusted to be optimal for the pathogen and not for the biocontrol agent. The inventors sought to study the ability of the *S. plymuthica* A30 to protect potato plants from blackleg in a worst possible scenario. For this purpose, blackleg-susceptible potato cultivar Kondor were used and a high inoculum of *Dickeya* sp. was applied directly to potato tubers to ensure high blackleg incidences. The biovar *3 Dickeya* sp. type strain was used because this is highly virulent and requires relatively high temperature and high humidity conditions to facilitate the infection process. The soil used in the experiments was collected from the potato field in order to mimic the natural field conditions. Nevertheless, *S. plymuthica* A30 was able to significantly reduce blackleg and affect *Dickeya* sp. densities in inoculated plants.

### Symptom development in plants

Plants were visually inspected weekly for development of the symptoms, i.e. plants were assessed for non-emergence, wilting and chlorosis of leaves, black rot on the stem base, aerial stem rot, haulm desiccation and for plant death. In repeated greenhouse experiments, strain A30 reduced symptom expression by *Dickeya* by 100% and colonization of stems by biovar 3 *Dickeya* sp. by 97% after sequential inoculation of tubers by vacuum infiltration. Table 1 shows the accumulated results of three groups of 10 plants, destructively analyzed at 1, 7 and 28 days after inoculation, respectively.

**Table 1. Disease incidence of potato plants sequentially inoculated with Dickeya sp. and/or S. plymuthica and of water inoculated control plants in a greenhouse experiment. The counts represent the accumulated incidence monitored in three groups of 10 plants per treatment per time point (i.e. 1, 7, and 28 days after inoculation).**

| treatment ^{a} | No. tested ^{b} | No. positive ^{c} | Positive (%) |
|---|---|---|---|
| water | 30 | 0 | 0 |
| GFP-tagged *S. plymuthica* A30 | 30 | 0 | 0 |
| DsRed-tagged *Dickeya* sp. | 30 | 19 | 63 |
| IPO3012 sequential inoculation | 30 | 2 | 7 |

| | | | |
|---|---|---|---|
| ^{a} tubers were inoculated by vacuum infiltration with DsRed tagged *Dickeya* sp. IPO3012 and/or by treating tubers with a suspension of GFP-tagged *S. plymuthica* A30 during planting. Tubers were inoculated with water as a negative control. ^{b} total number of plant screened ^{c} total number of plant expressing disease symptoms (i.e. pre-emergence tuber rot, chlorosis and wilting of leaves, typical blackleg, plant death) | | | |

In plants inoculated with *Dickeya* sp. IPO3012, first symptoms appeared 7 days post inoculation, when shoots were ca. 5 - 7 cm and roots were ca. 8-12 cm. Infections with *Dickeya* sp. IPO3012 severely affected sprouting and development of treated plant. Sixty percent of the plants infected with *Dickeya* sp. in first and 20% of plants in the second experiment showed pre-emergence tuber rot, deteriorations of shoot and root growth and first typical blackleg symptoms starting to develop on young shoots (i. e. wilting and chlorosis of leaves, water lesion on the stem surface) at 7 days post inoculation. At 28 days post inoculation, 60% and 50 % of plants inoculated with *Dickeya* sp. IPO3012 in experiment 1 and 2, respectively showed typical blackening and soft rotting near the base of the stems. Pre-emergence rot and development of blackleg symptoms were significantly reduced in plants co-inoculated with GFP-tagged A30 and DsRed-tagged *Dickeya* sp. IPO3012 strains. In the first experiment at 7 days post inoculation, only 10% of co-inoculated plants showed pre-emergence tuber rot and deterioration of shoot growth, however no disease symptoms were observed in co-inoculated plants at 28 days post inoculation. In the second experiment none of the co-inoculated plants showed pre-emergence tuber rot and blackleg symptoms during the entire course of the experiment.

Water inoculated control plants showed no symptoms during the entire course of both experiments (data not shown).

### Colonization of potato plants by DsRed-tagged Dickeya sp. IPO3012 and GFP-tagged S. plymuthica A30 followed by epifluorescence stereo microscopy

To access knowledge about internal colonization of roots and stems, plant parts were also screened using epifluorescence stereo microscopy. For this, plant samples were collected at 28 days post inoculation. Eight roots with a length of at least 5 - 10 cm and 6 shoots (stems) were cut randomly from every inoculated and water-inoculated control plant. All samples were washed and sterilized before microscopic observations as described for NAt pour plating. Each root was cut into 2-3 cm and each stem into 0.5 cm long fragments. Fragments were embedded in NA, cooled down to 48 °C containing 40 µg ml⁻¹ of tetracycline and 200 µg ml⁻¹ of cycloheximide in petri dishes. After the medium had solidified the plates were sealed with parafilm and incubated for 1-2 days at 28 °C. Plant parts harboring the green and/or red fluorescent signal were counted under an epifluorescence stereo microscope (Leica Wild M32 FL4) equipped with a mercury high pressure photo-optic lamp (Leica Hg 50W/AC) and a GFP and RFP plus filters.The number of plant samples showing typical green and/or red fluorescence was recorded as positive and fraction of positive samples was calculated for each treatment (see Table 2). Upon co-inoculation *S. plymuthica* A30 was able to decrease the *Dickeya* sp. colonization incidence in roots and stems or even eradicate the pathogen from inoculated plants.

**Table 2. Incidence of potato roots or stems colonized by GFP-tagged S. plymuthica A30 and/or DsRed-tagged Dickeya sp. IPO3012 at 28 days post inoculation.**

| treatment ^{a} | plant part ^{b} | No. tested ^{c} | NO. GFP positive ^{d} | GFP positive (%) | No. DsRed positive ^{d} | **DsRed positive (%)** |
|---|---|---|---|---|---|---|
| Water | roots | 80 | 0 | **0** | 0 | **0** |
| | stems | 60 | 0 | **0** | 0 | **0** |
| GFP-tagged *S. plymuthica* A30 | roots | 80 | 67 | **84** | 0 | **0** |
| | stems | 60 | 49 | **82** | 0 | **0** |
| DsRed-tagged *Dickeya* sp. IPO3012 | roots | 36 | 0 | **0** | 31 | **86** |
| | stems | 27 | 0 | **0** | 19 | **70** |
| sequential inoculation | roots | 72 | 64 | **88** | 7 | **8** |
| | stems | 54 | 49 | **90** | 0 | **0** |

| | | | | | | |
|---|---|---|---|---|---|---|
| ^{a} potato plants were inoculated by vacuum infiltration with DsRed tagged *Dickeya* sp. IPO3012, by soil infestation with GFP-tagged *S. plymuthica* A30 or co-inoculated with both strains, negative control were water inoculated plants grown in non infested soil ^{b} eight roots and six stem cuts were individually analyzed per plant and per time point ^{c} total number of plant analyzed per treatment (n=10) ^{d} number of plant parts that were positive for a GFP or DsRed signal | | | | | | |

### Quantification of DsRed-tagged Dickeya sp. IPO3012 and GFP-tagged S. plymuthica A30 in potato tissues by pour plating

Population dynamics of GFP-tagged *S. plymuthica* A30 and DsRed-tagged *Dickeya* sp. IPO3012 in soil and in plant parts (i. e. tubers, shoots and roots) were examined by NA pour plating at different time points.

Plants were sampled 1, 7 and 28 days post inoculation. At each time point, 10 plants per treatment were sampled. Samples were randomly collected from each pot and separately suspended in 2 ml of 1/4 Ringer's buffer supplemented with 0.02% dieethyldithiocarbamic acid (DIECA) as an oxidant. Hundred µl of the undiluted, 10 and 100 times diluted samples were mixed with NA supplemented with tetracycline to a final concentration of 40 µg ml⁻¹ (NAt), pre-warmed to 48 °C, and poured into the wells of a 24-well plate (Greiner). After agar had solidified, plates were wrapped with parafilm and incubated at 28 °C for 24-48 h for development of bacterial colonies. Plates were screened for GFP and/or DsRed positive colonies in the same way as it was done for interaction of GFP-tagged *S. plymuthica* A30 and *Dickeya* sp. IPO3012 on potato slices using a epifluorescence stereo microscope.

Per plant, seed tuber was collected and processed separately. Tubers were washed with tap water to remove soil particles, sterilized in 70% ethanol for 1 min, washed three times with water for 1 min, incubated in 1 % sodium hypochloride (commercial bleach) for 4 min and finally washed three times with water for 4 min. Each tuber was crushed in an Universal Extraction Bag (BIOREBA) using a hammer. Extracts were diluted and pour plated as described for the soil samples.

Per plant, all shoots were collected and processed as a composite sample. Per plant, the total root system was processed. After external washing and sterilization of both stem (shoot) samples and total root system, extracts were prepared and pour plated in the same way as described for seed tubers.

### Bacterial populations in tubers (see figure 4A).

Relatively low populations (average ca. 10¹ cfu g⁻¹) of A30 were detected inside seed tubers at 1 days post inoculation. Populations increased in 7 days to 10² - 10³ cfu g⁻¹ and stabilized at this level till 28 days post inoculation. In co-inoculated plants, *S. plymuthica* A30 populations in tubers at 1 days post inoculation were on average 10³ cfu g⁻¹ but populations decreased to 10² cfu g⁻¹ at 28 days. After tuber vacuum infiltration relatively high *Dickeya* sp. populations of 10⁴ cfu g⁻¹ were detected at 1 days post inoculation. At 28 days post inoculation populations were slightly declined to on average 10³ cfu g⁻¹. In co-inoculated plants, *Dickeya* sp. populations were significantly decreased from 10⁴ cfu g⁻¹ at 1 days post inoculation to on average 1 cfu g⁻¹ or less at 28 days post inoculation.

### Bacterial populations in roots (see figure 4B).

Bacterial populations in roots were analyzed only at 7 and 28 days post inoculation, as at 1 days post inoculation no roots were formed yet. At 7 days post inoculation, *S. plymuthica* A30 was present inside roots at a density of 10² cfu g⁻¹. At 28 days post inoculation, the population increased to 10³ - 10⁴ cfu g⁻¹. In co-inoculated plants the population dynamics of A30 followed the same trend. In *Dickeya* sp. IPO3012 inoculated plants, low *Dickeya* sp. populations (average less than 10¹ cfu g⁻¹) inside roots were found at 7 days post inoculation. Populations increased slightly to 10¹ - 10² cfu g⁻¹ at 28 days post inoculation. In co-inoculated plants, no *Dickeya* sp. was detected in roots at 7 days post inoculation. At 28 days post inoculation, only very low populations on average 1 cfu g⁻¹ were detected.

### Bacterial populations in shoots (see figure 4C).

Bacterial populations in shoots were analyzed only at 7 and 28 days post inoculation, as at 1 days post inoculation no shoots were formed yet. At 7 days post inoculation, *S. plymuthica* A30 was already present inside shoots at a density of 10² cfu g⁻¹. At 28 days post inoculation, the population increased 10 times. In co-inoculated plants the population dynamics of A30 followed the same trend. At 7 days post inoculation, in co-inoculated plants low densities of *Dickeya* sp. IPO3012 were detected in shoots of 5 - 10 cfu g⁻¹. Populations increased to a density of 10² - 10³ at 28 days post inoculation. In co-inoculated plants on average less than 10 cfu g⁻¹ of *Dickeya* sp. was present at 7 days post inoculation and no *Dickeya* sp. IPO3012 was detected in stems at 28 days post inoculation.

### Sampling of potato plants for confocal laser scanning microscopy (CLSM) to show bacterial colonization

For microscopy, plant samples were collected at the same time points as for the NA pour plating; that is 1, 7 and 28 days after inoculation. Eight roots with a length of at least 5 - 10 cm and 3 shoots (stems) were cut randomly from every inoculated plant. All samples were washed and sterilized before microscopic observations as described for NA pour plating.

Each root was cut into 2-3 cm and each stem into 0.5 cm long fragments. Fragments were embedded in NA, cooled down to 48 °C containing 40 µg ml⁻¹ of tetracycline and 200 µg ml⁻¹ of cycloheximide in petri dishes. After the medium had solidified the plates were sealed with parafilm and incubated for 1-2 days at 28 °C. After this time plant samples were collected from agar plates, washed briefly in demineralized sterile water and examined under the CLSM microscope. To monitor bacterial populations on the root surface, four roots per plant were processed without surface sterilization and without embedding.

For visualization of the plant cells, a 405 nm (excitation) ultraviolet laser and a 450 nm emission filter was used. For excitation of the GFP and DsRed in bacterial cells a 495 nm and 532 nm blue and green laser were used, respectively. For GFP, a 505 nm and for DsRed a 610 nm emission filter was used, respectively. Photographs were taken with a Leica Digital System (Leica) combined with a CLSM microscope using 10x and 63x water immersion objectives.

Plant parts were analyzed 7 and 28 days post inoculation, with a CLSM at a magnification of 640 and 1000 times. Detailed studies on the localization of the GFP-tagged *S. plymuthica* A30 and DsRed-tagged *Dickeya* sp. IPO3012 showed that at 7 days post inoculation both bacteria species were present inside the vascular tissue of the roots in pith (in medulla and cortex) both intra- and intercellularly (see figure 5A). In stems, fluorescent bacteria were found in the vascular tissue inside and between xylem vessels and protoxylem cells (figure 5B).

At 28 days post inoculation, in *Dickeya* sp. inoculated plants, DsRed-tagged bacteria were present inside and between pith cells of roots and inside and between xylem vessels of stems (see figure 5A and 5B).

In plants inoculated with *S. plymuthica* A30, GFP-tagged bacteria were present inside and between parenchyma cells of roots and in xylem vessels of stems (see figure 5A and 5B).

A30 appears to outcompete *Dickeya* in internal tissues of seed tuber, roots and stems. *Dickeya* was present in plant tissue at 7 days after inoculation but not at 28 days after inoculation.

### Colonization of root surface by GFP-tagged S. plymuthica A30 followed by CLSM

The ability of GFP-tagged *S. plymuthica* A30 to colonize roots of potato plants were tested, by analyzing randomly selected roots from S. plymuthica A30 inoculated plants at 28 days after inoculation using CLSM. All roots of plants grown in A30 inoculated tubers were surfacially colonized by GFP-tagged S. *plymuthica* A30 (see figure 6). GFP-tagged bacteria occurred in clumps or patches present on the root surface on the longitudinal axis of the roots, interspersed by areas where bacteria were absent or in which only low densities were present (see figure 6). No difference in surface colonization was observed for small and/or big and/or lateral and main roots (see figure 6).

In none of water control plants GFP-tagged bacteria on root surface were detected (see figure 6).

The results of the microscopic examinations showed that strain A30 is an endophyte as well as a rhizosphere colonizer. The strain was found in both roots (see Figure 5A) and stems (see Figure 5B) at 7 days after inoculation. Relatively low but stable populations of A30 built up. The *S. plymuthica* A30 probably enters the plant tissue via the roots, although it cannot be excluded that the bacterium may also enter via sprouts or injured tuber periderm. The inventors have noted transportation of both A30 and *Dickeya* in vascular tissue.

| | | |
|---|---|---|
| **0-1** | **Form PCT/RO/134 (SAFE) Indications Relating to Deposited Microorganism(s) or Other Biological Material (PCT Rule 13bis)** | |
| 0-1-1 | Prepared Using | **PCT Online Filing Version 3.5.000.225 MT/FOP 20020701/0.20.5.20** |
| **0-2** | **International Application No.** | |
| **0-3** | **Applicant's or agent's file reference** | **P132853WO** |
| | | |
| **1** | **The indications made below relate to the deposited microorganism(s) or other biological material referred to in the description on:** | |
| **1-1** | **page** | **5** |
| **1-2** | **line** | **26** |
| **1-3** | **Identification of deposit** | |
| 1-3-1 | Name of depositary institution | **BCCM Belgian Coordinated Collections of Microorganisms** |
| 1-3-2 | Address of depositary institution | **Federal Public Planning Service Science Policy, 8, rue de la Science, B-1000 Brussels, Belgium** |
| 1-3-3 | Date of deposit | **19 November 2010 (19.11.2010)** |
| 1-3-4 | Accession Number | **BCCM LMG P-26170** |
| **1-5** | **Designated States for Which Indications are Made** | **All designations** |

### FOR RECEIVING OFFICE USE ONLY

| | | |
|---|---|---|
| **0-4** | **This form was received with the international application:** (yes or no) | yes |
| 0-4-1 | Authorized officer | Wallentin, Marko |

### FOR INTERNATIONAL BUREAU USE ONLY

| | | |
|---|---|---|
| **0-5** | **This form was received by the international Bureau on:** | **16 January 2012 (16.01.2012)** |
| 0-5-1 | Authorized officer | **WAGNER, Nathalie** |

## Claims

1. *Serratia plymuthica* strain A30, BCCM Deposit No. LMG P-26170.

2. *Serratia plymuthica* strain A30 as claimed in claim 1 and which is
(a) metabolically active; or
(b) which is in the form of a solid preparation, e.g. a freeze dried preparation.

3. A biological culture comprising *Serratia plymuthica* strain A30, BCCM Deposit No. LMG P-26170 and a solid or a liquid medium, or a fraction thereof.

4. A composition for biological control of plant disease comprising *Serratia plymuthica* strain A30, BCCM Deposit No. LMG P-26170 and an agriculturally or horticulturally acceptable diluent, carrier, filler or adjuvant.

5. A composition as claimed in claim 4, wherein the carrier is a solid; optionally a porous solid.

6. A composition as claimed in claim 4 or claim 5, further comprising one or more of a further biological control agent, an antibiotic, a herbicide, a pesticide, a fungicide, a plant growth substance, fertilizer, a rooting substrate, e.g. a compost; soil, or inert particulate substrate such as vermiculite.

7. A growth substrate for plants comprising *Serratia plymuthica* strain A30, BCCM Deposit No. LMG P-26170.

8. A growth substrate as claimed in claim 7 which is or contains one or more of compost, sand, soil or an inert particulate material.

9. A plant, plant part or plant tissue comprising exogenous and/or endogenous *Serratia plymuthica* strain A30, BCCM Deposit No. LMG P-26170.

10. A method of preventing or treating disease in plants comprising exposing a plant, plant part or plant tissue to *Serratia plymuthica* strain A30, BCCM Deposit No. LMG P-26170.

11. A method as claimed in claim 10, wherein prior to storing or planting, a plant tuber is exposed to the *Serratia plymuthica* strain.

12. A method as claimed in claim 10 or claim 11, wherein a shoot or shoot portion of a plant is exposed to the *Serratia plymuthica* strain.

13. *Serratia plymuthica* strain A30, BCCM Deposit No. LMG P-26170, for use as a biological control agent in prevention or treatment of potato blackleg wherein the potato blackleg is caused by a bacterial plant disease pathogen selected from *Dickeya* spp. and *Pectobacterium* spp.

14. Serratia plymuthica strain A30 BCCM Deposit No. LMG P-26170 for the use as claimed in claim 13, wherein the bacterial plant disease pathogen is *Dickeya* sp. biovar 3 strain.

## Revendications

1. Souche A30 de *Serratia plymuthica,* dépôt au BCCM n° LMG P-26170.

2. Souche A30 de *Serratia plymuthica* selon la revendication 1, et qui est
(a) métaboliquement active ; ou
(b) qui est sous la forme d'une préparation solide, par exemple une préparation lyophilisée.

3. Culture biologique comprenant la souche A30 de *Serratia plymuthica*, dépôt au BCCM n° LMG P-26170 et un milieu solide ou liquide, ou une fraction de celle-ci.

4. Composition pour le contrôle biologique d'une maladie végétale comprenant la souche A30 de *Serratia plymuthica*, dépôt au BCCM n° LMG P-26170 et un diluant, un véhicule, une charge ou un adjuvant acceptable de manière agricole ou horticole.

5. Composition selon la revendication 4, dans laquelle le véhicule est un solide ; éventuellement un solide poreux.

6. Composition selon la revendication 4 ou la revendication 5, comprenant en outre un ou plusieurs d'un agent de contrôle biologique supplémentaire, un antibiotique, un herbicide, un pesticide, un fongicide, une substance de croissance végétale, un fertilisant, un substrat d'enracinement, par exemple un compost ; de la terre, ou un substrat particulaire inerte tel que de la vermiculite.

7. Substrat de croissance pour plantes comprenant la souche A30 de *Serratia plymuthica*, dépôt au BCCM n° LMG P-26170.

8. Substrat de croissance selon la revendication 7, qui est ou contient un ou plusieurs parmi le compost, le sable, la terre ou un matériau particulaire inerte.

9. Plante, partie de plante ou tissu de plante comprenant la souche A30 de *Serratia plymuthica*, dépôt au BCCM n° LMG P-26170 exogène et/ou endogène.

10. Procédé de prévention ou de traitement de maladie chez les plantes consistant à exposer une plante, une partie de plante ou un tissu de plante à la souche A30 de *Serratia plymuthica*, dépôt au BCCM n° LMG P-26170.

11. Procédé selon la revendication 10, dans lequel avant de stocker ou de planter, un tubercule végétal est exposé à la souche A30 de *Serratia plymuthica.*

12. Procédé selon la revendication 10 ou la revendication 11, dans lequel une pousse ou une partie de pousse d'une plante est exposée à la souche A30 de *Serratia plymuthica.*

13. Souche A30 de *Serratia plymuthica*, dépôt au BCCM n° LMG P-26170, pour une utilisation en tant qu'un agent de contrôle biologique dans la prévention ou le traitement de la jambe noire de la pomme de terre, la jambe noire de la pomme de terre étant causée par un pathogène bactérien de maladie végétale choisi parmi *Dickeya* spp. et *Pectobacterium* spp.

14. Souche A30 de *Serratia plymuthica*, dépôt au BCCM n° LMG P-26170, pour une utilisation selon la revendication 13, dans laquelle le pathogène bactérien de maladie végétale est la souche 3 de *Dickeya sp.* Biovar.

## Patentansprüche

1. *Serratia plymuthica*-Strang A30 mit der BCCM-Hinterlegungsnummer LMG P-26170.

2. *Serratia plymuthica*-Strang A30 nach Anspruch, für den folgendes gilt:
(a) er ist metabolisch aktiv; oder
(b) er ist in Form einem festen Präparat vorgesehen, wie etwa einem gefriergetrockneten Präparat.

3. Biologische Kultur, umfassend einen *Serratia plymuthica*-Strang A30 mit der BCCM-Hinterlegungsnummer LMG P-26170 und ein festes oder ein flüssiges Medium oder einen Teil davon.

4. Zusammensetzung zur biologischen Bekämpfung einer Pflanzenkrankreit, umfassend einen *Serratia plymuthica*-Strang A30 mit der BCCM-Hinterlegungsnummer LMG P-26170 und ein landwirtschaftlich oder gärtnerisch zulässiges Verdünnungsmittel, Trägermittel, Füllmittel oder Hilfsmittel.

5. Zusammensetzung nach Anspruch 4, wobei das Trägermittel ein Feststoff ist, optional ein poröser Feststoff.

6. Zusammensetzung nach Anspruch 4 oder Anspruch 5, wobei diese ferner eines oder mehrere der folgenden umfasst: ein weiteres biologisches Bekämpfungsmittel, ein Antibiotikum, ein Herbizid, ein Pestizid, ein Fungizid, eine Pflanzenwachstumssubstanz, ein Düngemittel, eine Wurzelbildungssubstanz, z.B. ein Mischdünger; Erde oder ein inertes partikuläres Substrat wie etwa Vermiculit.

7. Wachstumssubstrat für Pflanzen, das einen *Serratia plymuthica*-Strang A30 mit der BCCM-Hinterlegungsnummer LMG P-26170 umfasst.

8. Wachstumssubstrat nach Anspruch 7, das eines oder mehrere von Mischdünger, Sand, Erde oder eine inerte partikuläre Substanz ist oder enthält.

9. Pflanze, Pflanzenteil oder Pflanzengewebe, das einen exogenen und/oder endogenen *Serratia plymuthica*-Strang A30 mit der BCCM-Hinterlegungsnummer LMG P-26170 umfasst.

10. Verfahren zum Verhindern oder Behandeln einer Erkrankung von Pflanzen, umfassend das Aussetzen einer Pflanze, eines Pflanzenteils oder von Pflanzengewebe einem *Serratia plymuthica*-Strang A30 mit der BCCM-Hinterlegungsnummer LMG P-26170.

11. Verfahren nach Anspruch 10, wobei vor der Lagerung oder dem Pflanzen eine Pflanzenknolle dem *Serratia plymuthica*-Strang ausgesetzt wird.

12. Verfahren nach Anspruch 10 oder Anspruch 11, wobei ein Steckling oder ein Stecklingsteil einer Pflanze dem *Serratia plymuthica*-Strang ausgesetzt wird.

13. *Serratia plymuthica*-Strang A30 mit der BCCM-Hinterlegungsnummer LMG P-26170 zur Verwendung als ein biologisches Bekämpfungsmittel zur Prävention oder Behandlung von Kartoffel-Schwarzbeinigkeit, wobei die Kartoffel-Schwarzbeinigkeit verursacht ist durch ein bakterielles Pflanzenkrankheitspathogen, das ausgewählt ist aus *Dickeya* spp. und *Pectobacterium* spp.

14. *Serratia plymuthica*-Strang A30 mit der BCCM-Hinterlegungsnummer LMG P-26170 zur Verwendung nach Anspruch 13, wobei das bakterielle Pflanzenkrankheitspathogen ein *Dickeya* sp. Biovar 3-Strang ist.
